# EUROPEAN PATENT APPLICATION

(11) **EP 2 677 033 A2**
(43) Date of publication of application: **25.12.2013**
(21) Application number: 12726722.7
(22) Date of filing: 17.02.2012
(51) Int. Cl.: C12N 15/63

(54) **METHOD FOR GENETICALLY MODIFYING ACIDOPHILIC BACTERIA AND CONSTRUCTING A TRANSFORMATION VECTOR**

(30) Priority: 18.02.2011 BR PI1100418
(71) Applicant: Vale S.A., Cep: 20030-000 Rio de Janeiro - RJ (BR); Universidade Estadual Paulista Julio De Mesquita Filho - UNESP, 01049 - 010 Sao Paulo - SP (BR); Universidade Estadual De Campinas - Unicamp, 13083-970 Campinas - SP (BR)
(72) Inventor: CHRIST, Ana Paula Guarnieri, 13083-970 Campinas, SP (BR); ALEXANDRINO, Fabiana, CP 6010 Campinas, SP (BR); GARCIA JR., Oswaldo, 14801-970 Araraquara, SP (BR)
(74) Representative: EP&C
(86) International application number: PCT/BR2012/000045
(87) International publication number: WO 2012/116422

(57) **Abstract**

The present invention describes a process for engineering acidophilic chemolithotrophic bacteria by means of electroporation. The process is capable of engineering a bacterial line with a transformation vector called pAF vector, which contains a vegetative replication origin capable of provide the vector with the ability to self-replicate within the bacterium without changing its natural physiologic conditions and capable of enabling the use of such bacteria engineered according to the invention in the bioleaching process of ores that are sulphated of copper, gold, uranium, nickel, zinc, cobalt, without limitation.

## Description

### FIELD OF THE INVENTION

The present invention relates to a process for genetically engineering acidophilic chemolithotrophic bacteria without changing bacterial physiological properties so the new engineered strain can be capable of surviving and multiplying in its natural habitat.

The present invention also relates to a process for treating the cells of acidophilic chemolithotrophic bacteria, which is capable of transiently permeabilizing bacterial cell membrane in such a way the membrane becomes permissive to the entry of exogenous genetic material. In addition, the object of this invention is the process for inserting genetic material of interest into the strains of acidophilic chemolithotrophic bacteria and also for constructing a vector which is capable of self-replicating when inserted into cells of acidophilic chemolithotrophic bacteria.

### BACKGROUND OF THE INVENTION

Bacteria of the *Acidithiobacillus* genus are acidophilic, autotrophic and chemolithotrophic, that is, these bacteria live in acidic pH conditions, 0-4, their carbon source is CO₂, and their energy source is inorganic compounds. Two species of this genus are important and commonly used in biomining: *Acidithiobacillus ferrooxidans* and *Acidithiobacillus thiooxidans.*

The species *Acidithiobacillus ferrooxidans* represents gram-negative, nonpathogenic bacteria, with optimal growing around 30°C and pH around 2.0, and has been used as the standard microorganism for the bioleaching process studies (Holmes et al., 2008, BMC Genomics 9:597).

As mentioned above, *Acidithiobacillus ferrooxidans* is one of the most used microorganisms for the bacterial leaching of metals, process known as bioleaching. During the bioleaching process, microbial metabolism causes the solubilization of the ores that have reduced forms of sulfur and iron in its composition. This solubilization can occur both directly, via enzymatic attack, or indirectly, via production of H₂SO₄ and Fe³⁺ by bacterial metabolism.

Bioleaching is a consolidated technique in metallurgical and mining industry and it is successfully applied for the treatment of refractory gold ores and for the recovery of copper in marginal ores and tailings. Bioleaching is defined as a method for the solubilization of metals from complex matrices in acidic medium employing the direct or indirect action of microorganisms (Rawlings 2005, Microb. Cell. Fact. 4(1): 13). Bioleaching is direct when microorganisms act on the metal or on its counterion, releasing an ion of the metal of interest into the solution in both cases. On the other hand, bioleaching is indirect when the microorganism generates chemical conditions that accelerate and promote metal solubilization.

Bioleaching is responsible for approximately 10% of worldwide production of copper and is particularly important for the recovery of metals from low-content ores, for which the extraction by standard process like pyrometallurgy would not be economically viable. Thus, the application of bioleaching for the recovery of metals from low-content ores was shown to be an economically and environmentally viable alternative, because it does not require high power consumption and, in comparison to pyrometallurgy, it can be considered a less polluting strategy (Jonhson & Rawlings, 2007, Microbiology 153: 315-24).

The species of the genus *Acidithiobacillus* are used in bioleaching processes, and the species *Acidithiobacillus ferrooxidans* favors the oxidation of Fe²⁺ into Fe³⁺, employing oxygen as a receptor of electrons. The Fe³⁺ that is generated is an agent with higher oxidative potential and it is able to oxidize sulphides and any compound that is necessary to oxidize. Despite the large use of *Acidithiobacillus ferrooxidans* in the bioleaching process, its slow growth associated to its sensitivity to the ion chloride, and to heavy metals like As³⁺, Hg²⁺ and Ag⁺ has limited a broader application of this technology.

In light of the importance of *Acidithiobacillus ferrooxidans* for the bioleaching process, the ability to genetically engineer this mechanism becomes highly important. Employing an effective method to transform the bacterial cells can improve the oxidation activity, increase the resistance to toxic compounds, accelerate the growth and incorporate properties of interest.

Genetic engineering of *Acidithiobacillus sp.* has been shown to be very complex. However, due to the relevance of *Acidithiobacillus ferrooxidans* in the bioleaching process, the standardization of processes enabling to genetically engineer this species, improving its growth and oxidation conditions, as well as the incorporation of interesting properties, like the resistance to toxic compounds, is critical.

Basic information on the molecular genetics of *Acidithiobacillus ferrooxidans* have been collected for years. Several authors describe attempts to promote the transference of genetic material to *Acidithiobacillus spp.* using distinct approaches, such as, for example, the use of systems to conjugate acidophilic strains and *E. coli,* and mainly using the electroporation technique.

The culture of *Acidithiobacillus* bacteria is complex, since these bacteria must be maintained under extremely acid pH conditions. This characteristic makes the process for the engineering of these microorganisms more difficult.

The standardization of a reproducible genetic engineering process for *Acidithiobacillus ferrooxidans* and for the other acidophilic chemolithotrophic microorganisms that are used in the bioleaching process, such as, for example, *Acidithiobacillus thiooxidans, Acidithiobacillus caldus, Acidithiobacillus prosperus, Leptospirillum ferrooxidans, Sulfobacillus thermosulfidooxidans, Sulfolobus metallicus, Sulfolobus acidocaldarius, Acidianus brierleyi, and Acidianus infernus,* has been challenging and once in a while it is not that promising.

Roberto and Bruhn (Roberto and Bruhn; 1992, Geomicrobiology Journal (10(3-4), 249-55) described that the use of electroporation is limited for the engineering of acidophilic bacteria and that the conjugation technique seems to be more applicable.

Kusano and colleagues (Kusano et al., 1992, J Bacteriol. 174: 6617-6623) described the electroporation of 30 strains of *Acidithiobacillus ferrooxidans* as a vector carrying a mercury resistance gene *(operon mer).* Only one of these strains was engineered and the reproducibility of the technique was very low, and no other group was able to reproduce the process these authors described.

In 1995, English and colleagues (English et al., 1995, Appl. Environ. Microbiol. 61: 3256-3260) described an electrotransformation trial with *Thiobacillus neapolitanus* and a vector carrying the carboxysome replicon of *Thiobacillus intermedius.* Despite the electroporation results were positive, the authors could not show protein expression, which would be an indication of lack of functionality for the vector.

Studies have been identified showing interest on engineering and/or identification of genetic factors in acidophilic and chemolithotrophic microorganisms with the intention of improving the activity of these microorganisms in bioleaching (Bruscella et al; 2007, Microbiology 153 (1): 102-10; Farah et al; 2005, Appl. and Environ. Microbiol. 71(11): 7033-7040).

Studies based on the conjugation system generated four publications describing the genetic transference among strains of *Escherichia coli* and *Acidithiobacillus spp* (Yankofsky et al., 1983, J. Bacteriol. 153: 652-657; Jin et al., 1992, Appl. Environ. Microbiol. 58: 429-430; Liu et al., 2000, J. Bacteriol. 182: 2269-2276 and van Zyl et al., 2008, Appl. Environ. Microbiol. 74: 5686-5694). Nonetheless, only the van Zyl and colleagues study was shown to be a technique efficiently able to generate a null mutant from an *Acidithiobacillus caldus* strain, however this sort of approach is not yet described for lines of *Acidithiobacillus ferrooxidans.*

Recently, the US patent application number US 2009/0035864 described an engineering process for *Acidithiobacillus spp.* based on the modification of bacterial growth conditions. The group reports that cultures of *Acidithiobacillus spp.* adapted to pH conditions within 5-7 are liable to engineering by means of any known process, especially by electroporation. However, taking into consideration that the optimal growth pH for *Acidithiobacillus ferrooxidans* is within 1.5-2.5, the adaptation of this organism to a higher pH (pH within 5-7) represents a severe change to the bacterial physiology. Any strain that is engineered under changed physiological conditions gets its metabolism adapted to specific conditions, and thus, this new microorganism can lose the ability to grow under natural conditions. According to Suzuki (Suzuki, 2001, Biotechnology Advances 19: 119-132), the microorganisms that act in ore bioleaching / bio-oxidation processes need specific characteristics in order to have a good performance, such as the possibility of acting under an expanded temperature (30° to 70°C) range, an acidic pH range (1.8-2.2) and a high ratio of Fe³⁺ to Fe²⁺ ions. Thus, native bacteria have some advantages over collection strains, once they are already adapted to weather conditions, to mineralogy, and to the chemical composition of the ores to be treated.

In summary, to date, there is no known and reproducible process through which it is possible to engineer *Acidithiobacillus ferrooxidans* cells without changing their physiological properties. The same can be applied to the other acidophilic chemolithotrophic microorganisms employed in the bioleaching process, thus not being limited to the bacterium *Acidithiobacillus ferrooxidans.*

In addition, there are few published papers on this area and most of them show results that are inconclusive or irreproducible, since the genetic handling of these microorganisms is difficult. In addition to the slow growth and the small number of cells, the culture of such microorganisms in solid media is difficult, thus making unfeasible the conduction of several trials.

Therefore, as *Acidithiobacillus ferrooxidans* is the most commonly used microorganism in the bioleaching processes, the development of a genetic engineering process for *Acidithiobacillus ferrooxidans,* without changing its natural physiology, is of utmost importance for the bioleaching field. Aiming at obtaining *Acidithiobacillus ferrooxidans* cells engineered for better bioleaching, a process was developed for engineering the permissive line of *Acidithiobacillus ferrooxidans,* without changing the natural species physiology, with the use of a functional vector that is capable of self-replication within the cell.

### DESCRIPTION OF THE INVENTION

The present invention relates to a process for engineering acidophilic chemolithotrophic bacteria without changing natural bacterial physiological properties so the new engineered strain can be capable of surviving and multiplying in its natural habitat.

According to this invention, the engineering process consists of the following steps: (a) obtainment of *Acidithiobacillus ferrooxidans* competent cells by means of changing the permeability of the cell membrane; (b) construction of a vector that is capable of self-replication when inserted into *Acidithiobacillus ferrooxidans;* (c) transformation of *Acidithiobacillus ferrooxidans* cells, previously induced to the transient state of competence using electroporation technique.

A second feature of this invention deals with the construction of a self-replicating vector, that is, a vector containing DNA sequences which make it functional.

In addition, this invention also comprises one process for the treatment of *Acidithiobacillus ferrooxidans* cells, which is capable of transiently permeabilizing the cell membrane of said bacterial cell in order to become permissive and allow the entry of exogenous genetic material, for example, vectors.

Another aspect of this invention consists of the importance that the origin of replication is inserted into the vector. This origin must allow the replication of plasmid within the *Acidithiobacillus ferrooxidans* cells.

### BRIEF DESCRIPTION OF THE FIGURES

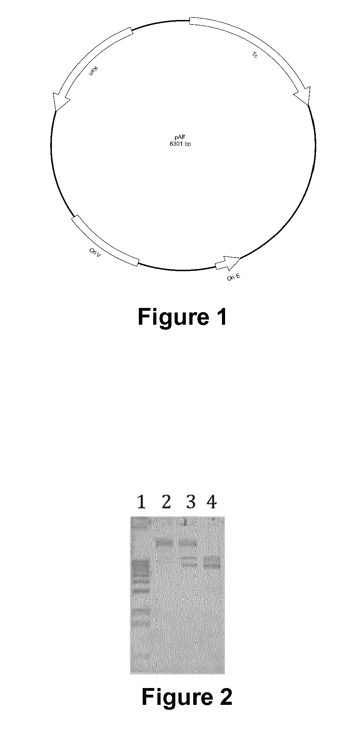

Figure 1 shows the pAF vector diagram, highlighting the essential characteristics which make it functional in *Acidithiobacillus spp.* OriE - allows the replication of plasmid in *Escherichia coli,* which is required for subcloning; Tc-tetracycline resistance gene; Kan - kanamycin resistance gene; OriV - allows the replication of plasmid in *Acidithiobacillus spp.*
Figure 2 shows a 1% agarose gel with the recovery of pAF vector from rLRpAF: (1) molecular weight marker 1 kg plus (Invitrogen^{®}); (2) Isolated plasmid DNA from rLRpAF; (3) DNA from a culture of *Acidithiobacillus ferrooxidans* LR non-recombinant; and (4)1 µg of DNA from pAF (positive control).

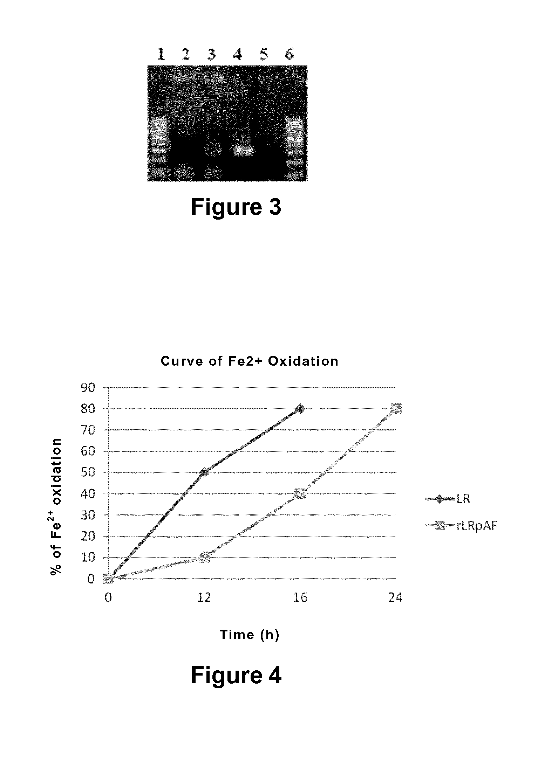

Figure 3 shows the amplification of OriV from rLRpAF: (1) molecular weight marker 100 pb (Invitrogen^{®}); (2) PCR from a culture of *Acidithiobacillus ferrooxidans* LR; (3) PCR from a culture of rLRpAF; (4) PCR from a culture of pAF DNA; (5) PCR without sample (negative control) and (6) molecular weight marker 100 pb (Invitrogen^{®}).
Figure 4 shows a chart of the curve of Fe²⁺ oxidation by engineered (rLRpAF) and non-engineered *Acidithiobacillus ferrooxidans.*

### DETAILED DESCRIPTION OF THE INVENTION

The present invention relates to a process for the engineering of acidophilic chemolithotrophic bacteria, namely the LR line of *Acidithiobacillus ferrooxidans.* Such engineering process employs a vector which is characterized by containing a vegetative replication origin, which confers to vector the ability of self-replicating within the engineering cell.

Of note, this invention is not limited to LR line of *Acidithiobacillus ferrooxidans,* neither to the *Acidithiobacillus* bacteria. This invention may also be employed for the engineering of other acidophilic chemolithotrophic microorganisms, whether heat-tolerant or not, such as, for example, without limitation, *Acidithiobacillus thiooxidans, Acidithiobacillus caldus, Acidithiobacillus prosperus, Leptospirillum ferrooxidans, Sulfobacillus thermosulfidooxidans, Sulfolobus metallicus, Sulfolobus acidocaldarius, Acidianus brierleyi,* and *Acidianus infernus.*

The engineering of bacteria is a process through which the plasmid DNA (vector) is introduced into the transiently permeable bacterial cell for the purposes of introducing genetic material of interest.

According to this invention, the bacterial engineering process occurs without changing the natural physiological properties of the bacteria, in such a way the engineered strain can be capable of surviving and multiplying in its natural environment.

According to this invention, the engineering process consists of the following steps: (a) obtainment of competent cells of acidophilic chemolithotrophic bacteria by means of changing the permeability of the cell membrane; (b) construction of a vector that is capable of self-replication when inserted into cells of acidophilic chemolithotrophic bacteria; (c) transformation of cells of acidophilic chemolithotrophic bacteria, previously induced to the transient state of competence using electroporation technique.

According to this invention the term "competent cells" encompasses cells that are permissive to the entry of exogenous material.

According to the present invention, one of the successful requirements for the engineering process of *Acidithiobacillus ferrooxidans,* as well as of the other acidophilic chemolithotrophic microorganisms, is the development of vectors capable of self-replicating within bacterial cells.

For the purposes of obtaining a functional vector, some characteristics have been explored. Some plasmids, known to be broad-host-range plasmids, are capable of self-replicating within several species of bacteria, being known as promiscuous plasmids. Broad-host-range plasmids, especially those pertaining to the incompatibility group of gram-negative bacteria (IncQ and IncP plasmids), were the most studied, exhibiting characteristics that are suitable for the development of a host-vector system for those lines of bacteria having no natural plasmids or for which there are no plasmids described yet.

According to this invention, a common and important vector characteristic is the presence of a DNA sequence referred to as vegetative replication origin (OriV), a sequence that is responsible for conferring the self-replication ability to the material that is inserted into several types of gram-negative bacteria.

Rawlings and colleagues (Rawlings et al., 1989, J. Bacteriol. 171; 2735-2739) isolated a replication region from a broad-host-range plasmid of *Acidithiobacillus ferrooxidans* (pTF-FC2). This region is largely similar to the OriV of a IncQ plasmid of *Escherichia coli.* Cloning of this DNA sequence from *Acidithiobacillus ferrooxidans* enables the vector to replicate on the GW125a line of *Escherichia coli* (DNA polymerase-deficient line), suggesting that a plasmid containing a similar sequence would be able to self-replicate within gram-negative lines of bacteria, including *Acidithiobacillus ferrooxidans.*

In the present invention, we can note that the 200 pb OriV amplified from a commercial transposon (EZ-Tn5™) exhibited 44.05% of similarity to the sequence found in the PTF-FC2 plasmid described by Rawlings and colleagues (Rawlings et al., 1989, J. Bacteriol. 171: 2735:2739), and 100% of similarity to the IncP and the promiscuous plasmids R18, R68, RK2, RP1 and RP4 from *E. coli* (Peng et al., 1994, J. Bacteriol. 176:2892-2897). Thus, the insertion of this OriV, taken from the EZ-Tn5™ transposon, enables the construction of a vector that is very likely to replicate within *Acidithiobacillus ferrooxidans.* Therefore, the approach employed in this invention was shown to be efficient, since the pAF vector was capable of replicating with *Acidithiobacillus ferrooxidans* cells, as we can see in figures 2 and 3. And the commercially available promiscuous origin of replication enables the construction of vectors for genetically engineering lines of *Acidithiobacillus* and other acidophilic chemolithotrophic microorganisms in case the lines to be engineered have no natural plasmids.

The construction of a functional vector must encompass some basic elements: (1) origin of replication from *E. coli* (Ori E), enabling the conduction of subcloning in lines of *E. coli;* (2) an adequate promoter, preferably lac P (promoter of the gene encoding β-galactosidase); (3) antibiotic resistance genes, preferably ampicillin, kanamycin or tetracycline, in order to promote the selection of engineered cells; (4) a multiple cloning site (MCS) for the insertion of genes or fragments of interest; and finally (5) a reporter gene encoding, for example, an enzyme, like β-galactosidase, or a green fluorescent protein as secondary marker.

According to this invention, the vector was constructed using molecular biology techniques (Sambrook et al., 1989, Molecular Cloning: A Laboratory Manual), as described in Example 1.

Once the vector has been constructed, the next step, according to this invention, consists of the engineering of the LR line of *Acidithiobacillus ferrooxidans* used as example.

The genetic engineering of species from the genus *Acidithiobacillus* and of other acidophilic chemolithotrophic bacteria employed in the bioleaching processes has been particularly challenging. One of the major issues contributing toward said difficulty is the substrate where those bacteria are cultured. The fact that the substrate is a mixture of inorganic salts adjusted to a very low pH range interferes with the steps required for the genetic handling of these microorganisms. The optimal way is that genetic engineering should occur in such a way it does not change the physiology of the bacteria, thus avoiding problems like their growth and physiological readaptation and plasmid DNA stability.

Thus, for the purpose of resolving the existing problem, the present invention describes a genetic engineering process which enables the engineering of acidophilic chemolithotrophic bacteria, like *Acidithiobacillus ferrooxidans,* via electroporation, preserving their culture, following the engineering process, at the same conditions of bacterial natural habitat, that is, same pH and temperature conditions, thus not changing their natural physiological state.

The genetic engineering process is composed of six important steps: (1) standardization of inoculate growth phase so the bacteria can be more susceptible to the permeabilization of its membrane; (2) permeabilization or weakening of bacterial cell wall, resulting in the obtainment of competent cells; (3) application of electric pulses; (4) incorporation of plasmid DNA with genes of interest using appropriate vectors; (5) inoculate and recovery; and (6) selection of engineered cells.

According to the present invention, the optimal growth phase is that phase when the culture of cells reaches the range of 50-80% of Fe²⁺ oxidation in the media, preferably between 60-70%, once the cells within this range were noted to be more permissive to the following process steps.

Once cultured cells reach the phase of optimal growth, cells are washed with saline solution in order to acclimatize them to a solution with pH conditions appropriate for the preservation of the stability of reagents and DNA which will be used in the following steps. This change to the substrate is crucial so the vector used for the engineering step and the antibiotic used for the selection step be not denatured or degraded by the low pH conditions of the media. Following the wash with saline solution, cultured cells are incubated with a cell-permeabilizing agent, generating electrocompetent cells of acidophilic chemolithotrophic bacteria. The most common electroporation processes use glycerol or glycine as cell-permeabilizing agents. However, during the trials, glycerol was found to be toxic for *Acidithiobacillus ferrooxidans* cells, promoting the cytolysis even at low concentrations (0.5%). Nonetheless, the incubation of cells in solutions containing 0.5-3.0% glycine in mass/volume, preferably from 1.0-2.0% in mass/volume, at an optimal concentration of 1.0-1.5% in mass/volume, was shown to be efficient for the permeabilization of cell wall and did not cause cytolysis, generating electrocompetent cells, permissive to the incorporation of DNA.

In the next process step, the incubation of electrocompetent cells takes place *(Acidithiobacillus ferrooxidans* LR cells, previously treated with glycine), with 1-5 µg of DNA (Vector pAF), and is followed by the application of the pulse generated by the equipment BioRad Gene® Pulser, responsible for promoting the formation of temporary pores in cell membrane. The vector penetrates the cell through these transient pores which have been opened during the electric pulse under optimal conditions, which have been determined as a result of several attempts. According to the present invention, the optimal electric pulse conditions are: Capacitance of 25 µF; Resistance of 200Ω; Tension between 1.0-2.0 kV, being the preferred optimal tension 1.0-1.5 kV; pulse duration 2-5 milliseconds.

Aiming at recovering and stabilizing the cell membrane after the electric pulse, the cells of Acidithiobacillus ferrooxidans were incubated for 1 hour in an appropriate culture media, such as T&K, supplemented with FeSO₄.7H₂O, pH within 1.5-2.5, preferably pH 1.8. This step allows the electroporated cells to recover in the optimal culture media, resuming their natural physiological conditions.

The T&K culture media, developed by Tuovinen & Kelly (1973), has been used with minor changes; it represents one of the specific culture media for species of *Acidithiobacillus ferrooxidans.* The T&K media is formed by the mixture of two solutions A and B at 4:1 ratio, respectively. The Solution A is composed of 0.4 g/l of K₂HPO₄.3H₂O, 0.4 g/l of MgSO₄.7H₂O, 0.4 g/l of (NH₄)₂SO₄, and Solution B is composed of only 33.4 g/l of FeSO₄.7H₂O.

After stabilized, the engineered cells were subjected to the selection step, the purpose of this step is selecting the cells that preserved the incorporated plasmid DNA, that is, the engineered (recombinant) cells. Selection is conducted in the presence of a selective agent and, particularly in this case, the selective agent is an antibiotic, and engineered cells are resistant to this antibiotic. In the case of this invention, the engineered cells are resistant to the antibiotics kanamycin.

After the recovery period, the inoculate is washed again for several times with saline solution in order to balance the cells in a substrate suitable for the treatment with the antibiotic, since most of known antibiotics are degraded with the pH range below 4.0. Then, the cells were incubated for a period of at least 2 hours and not longer than 8 hours, being 4 hours the preferred incubation period, in saline solution containing 50 µg/ml of kanamycin. The 4-hour preferred incubation period has been previously standardized and ensures that all cells that survive to this selection contains the kanamycin-resistant gene, that is, they are recombinant / engineered cells, which incorporated the pAF vector, and were consequently identified as rLRpAF cells. It is important to note that the selection agent is not limited to kanamycin.
After selection, the rLRpAF cells are inoculated into a culture medium, specifically into a T&K medium supplemented with FeSO₄.7H₂O, with a pH varying from 1.5 to 2.5, preferably with a pH of 1.8, in order to obtain the cell mass required for the experiments confirming the presence of the pAF vector. Optionally, the recombinant / transformed cells may be plated in a solid T&K medium with a pH between 1.5 and 25, preferably with a pH of 1.8, kept at 30°C for 10 to 20 days, with the colonies that develop corresponding to the rLRpAF cells.
The examples of the procedures conducted in this invention are set forth below and must not be considered as a limitation, but rather as an illustration of the invention. It is stressed that the *Acidithiobacillus ferrooxidans* LR lineage was used as an experimental lineage, and that the proposed process is not limited to the transformation of just this lineage, and may be applied to any acidophilic chemolithotrophic microorganism lineage, thermotolerant or not, such as for example and not limited to: *Acidithiobacillus thiooxidans, Acidithiobacillus caldus, Acidithiobacillus prosperus, Leptospirillum ferrooxidans, Sulfobacillus thermosulfidooxidans, Sulfolobus metallicus, Sulfolobus acidocaldarius, Acidianus brierieyi* and *Acidianus infemus.*
We stress that all the units mentioned in the description of this invention comply with the International System of Units.

### EXAMPLES

### Example 1: Construction of the pAF Vector

The commercial pBR322 vector (Invitrogen®) was used as a skeleton for the
construction of the pAF vector. The origin of vegetative replication (OriV) was previously amplified through a commercial transposon (EZ-Tn5™) and inserted in a pGEM-T subcloning vector. Subsequently, the OriV fragment was removed from the pGEM-T OriV at the Cla I and *Hind* III restriction sites and this fragment was cloned in pBR322 at the same restriction sites. The kanamycin resistance gene (Kan) was removed from the pET28a vector, sold by the Novagen company, and subcloned at an MCS site (multiple cloning site) on the pUC19 vector between the *Hind* III and *Sal* I restriction sites. The pBR3220riV was then digested with the *Hind* III and *Sal* I enzymes for the insertion of the kanamycin resistance gene, generating the pBR3220riV-Kan vector. The vector resulting from this construction, called pAF, presents approximately 6301 pb and its map is shown in Figure 1.
In order to support this example, the DH5a lineage of *Escherichia coli* was transformed with the pAF vector, using o CaCl2 process. The transformed colonies were selected in a solid LB culture medium containing 50 pg/mL of kanamycin. Widely used for bacteria growth and maintenance, the LB medium consists of 10 g/l of triptone, 5 g/l of yeast extract, 5 g/l of sodium chloride and 15 g/l of agar for solid culture mediums.
The kanamycin-resistant colonies were inoculated into a liquid medium containing 50 µg/l of kanamycin for subsequent plasmid extraction by mini-preparation. Using the PCR technique, amplification of different regions (OriV and kanamycin gene) of the plasmid was then undertaken, with the outcomes confirming that the isolated plasmid is the pAF vector constructed in compliance with this invention.

### Example 2: Obtaining competent Acidithiobacillus ferrooxidans LR cells

The Acidithiobacillus ferrooxidans LR cells were grown in a liquid T&K medium (K2HPO4.3H2O, 0.4 g/l; MgSO4.7H2O, 0.4 g/l; (NH4)2SO4, 0.4 g/l), supplemented with FeSO4.7H2O (33.4 g/l), pH of 1.8 adjusted with sulfuric acid, and incubated for 18 hours stirred at 250 rpm at 30°C. Cell growth was interrupted when the culture reached between 50% and 80% and Fe2+ oxidation in the medium (titrated with potassium dichromate - K2Cr207).

The cells were collected through filtration with 0.45 µm membrane filtration and washed several times with saline solution (NaCl 0.9%, in mass/volume, with Milli-Q water, pH 5.8-7.4). In order to obtain electrocompetent cells of *Acidithiobacillus ferrooxidans* LR, the biomass from a 500 mL culture was incubated in 1 mL of saline solution containing glycine 1-2%, in mass/volume, being the optimal concentration 1-1.5% of glycine, in mass/volume, for 30 minutes, under agitation (250 rpm) at 30°C. Next, the cells were collected and incubated in ice for 1 hour. Following incubation, the cells were washed twice with washing buffer (sodium phosphate 5 mM, magnesium chloride 1 mM, pH 5.8-7.4) and ultimately resuspended at 200 µl of electroporation buffer (1 M of sucrose, 3 mM of magnesium chloride, ph 5.8-7.4).

### Example 3: Electrotransformation of Acidithiobacillus ferrooxidans LR

The electrocompetent *Acidithiobacillus ferrooxidans* LR cells have been obtained as described in example 2. For each 45 µl of electrocomponent cells, 1 µg of plasmid DNA have been added (pAF vector, constructed according to this invention). This mixture was subjected to electroporation in 0.2 mm cuvettes stored in ice until pulse emission. Electroporation conditions were 1.0-2.0 kV, 200Ω for 2.4-5 milliseconds. Just after that, the cells were recovered in 1 mL T&K medium supplemented with FeSO₄.7H₂O (ph 1.8), and incubated for 1 hour under agitation (250 rpm) at 30 °C. Recovered cells were collected and washed twice with saline solution and inoculated in 2 mL saline solution containing 50 µg/ml kanamycin for 4 hours (under 250 rpm agitation and at 30 °C), aiming at inducing the expression of the gene encoding antibiotic resistance. After such selection, the cells were collected and inoculated in 10 mL of T&K media containing FeSO₄.7H₂O (pH 1.8), and incubated at 30 °C under 250 rpm agitation.

The electrotransformed (recombinant) cells of *Acidithiobacillus ferrooxidans* LR with the pAF vector were named rLRpAF. Of note, the LR line recombinant cells can be cultured in T&K media with pH 1.8, meaning that the process of this invention is suitable for engineering the bacterium without changing its physiological state.

### Example 4: Recovery of pAF vector from rLRpAF culture

The engineered cells of the bacterium *Acidithiobacillus ferrooxidans* with the pAF vector selected with kanamycin for 4 hours were inoculated into 500 ml of T&K media supplemented with FeSO₄.7H₂O (pH 1.8) until the culture reached 90% oxidation of Fe²⁺ in the media, aiming at obtaining sufficient cells for the extraction of plasmid DNA.

Plasmid DNA was isolated with the Invitrogen® Mini Prep kit and subjected to 1% agarose gel electrophoresis. Figure 2 shows a picture of 1% agarose gel containing a 1 kb plus molecular weight marker (Invitrogen^{®}), plasmid DNA isolated from rLRpAF, plasmid DNA isolated from one culture of *Acidithiobacillus ferrooxidans* LR and 1 µg of DNA of pAF vector (positive control). The presence of a band (pointed out by the arrow) shows that the recombinant LR line has the plasmid DNA present in the pAF vector.

An alternative media to test the presence of pAF vector in rLRpAF was the PCR technique for the region containing plasmid OriV. The expected size of amplified fragment was approximately 350 pb. The PCR reactions were conducted with cell mass obtained from rLRpAF and LR cultures. The amplification of pAF vector DNA was conduct as PCR positive control, and a blank reaction was run as negative control. The result is presented in Figure 3, which shows the amplification of rLRpAF OriV.

### Example 5: Curve of Fe²⁺ oxidation by engineered and non-engineered Acidithiobacillus ferrooxidans

Aiming at comparing the oxidation of Fe²⁺ by the engineered *Acidithiobacillus ferrooxidans* LR with pAF vector, and the non-engineered one, both the engineered and the non-engineered bacteria were inoculated in T&K media supplemented with FeSO₄.7H₂O (pH 1.8) and incubated under agitation (250 rpm) at 30 °C. As shown in Figure 4, rLRpAF shows a delay in Fe²⁺ oxidation in relation to the non-engineered line due to the time period the bacterium requires to be recovered from the engineering process. This assay shows that the engineered line is capable of oxidizing Fe²⁺.

## Claims

1. PROCESS FOR GENETIC ENGINEERING OF ACIDOPHILIC BACTERIA **characterized by** encompassing the following steps: (1) obtainment of competent *Acidithiobacillus ferrooxidans* cells: (2) constructing a vector that is capable of self-replication when inserted into *Acidithiobacillus ferrooxidans* cells; (3) transformation *Acidithiobacillus ferrooxidans* cells previously induced to the transient state of competence using electroporation technique.

2. PROCESS FOR GENETIC ENGINEERING OF ACIDOPHILIC BACTERIA according to claim 1, **characterized by** not changing the physiological properties of bacteria.

3. PROCESS FOR GENETIC ENGINEERING OF ACIDOPHILIC BACTERIA according to claim 1, **characterized by** a step for the obtainment of competent cells composed of the following steps: (a) standardization of inoculate growth phase; (b) filtration of cells; (c) permeabilization or weakening of bacterial cell wall in order to obtain electrocompetent cells.

4. PROCESS FOR GENETIC ENGINEERING OF ACIDOPHILIC BACTERIA according to claim 3, **characterized by** the standardization of the inoculate growth phase being the phase in which the inoculate reaches 50-80% of oxidation of Fe²⁺ in the media.

5. PROCESS FOR GENETIC ENGINEERING OF ACIDOPHILIC BACTERIA according to claim 4, **characterized by** the standardization of the inoculate growth phase having 60-70% of Fe²⁺ oxidation in the medium.

6. PROCESS FOR GENETIC ENGINEERING OF ACIDOPHILIC BACTERIA according to claim 3, **characterized by** a filtration step conducted with 0.45 µm membrane and successive washes with saline solution.

7. PROCESS FOR GENETIC ENGINEERING OF ACIDOPHILIC BACTERIA according to claim 6, **characterized by** a saline solution that is 0.9% NaCl, in mass/volume, of NaCl in water and a pH range of 5.8-7.5.

8. PROCESS FOR GENETIC ENGINEERING ACIDOPHILIC BACTERIA according to claim 3, **characterized by** a cell wall permeabilization step encompassing the following steps: (a) incubation of previously washed cells with solution of cell-permeabilizing agent; (b) successive washes of cells using washing buffer; (c) resuspension of cells in electroporation buffer.

9. PROCESS FOR GENETIC ENGINEERING OF ACIDOPHILIC BACTERIA according to claim 8, **characterized by** a permeabilizing agent that is glycine.

10. PROCESS FOR GENETIC ENGINEERING OF ACIDOPHILIC BACTERIA according to claim 9, **characterized by** glycine diluted into a saline solution comprising about 0.5-3.0%, in mass/volume, of glycine.

11. PROCESS FOR GENETIC ENGINEERING OF ACIDOPHILIC BACTERIA according to claim 10, **characterized by** a glycine solution comprising around 0.5-3.0%, in mass/volume, of glycine, at an optimal concentration of 1.0-2.0%, in mass/volume, of glycine.

12. PROCESS FOR GENETIC ENGINEERING OF ACIDOPHILIC BACTERIA according to claim 8, **characterized by** an incubation of cells with permeabilizing agent conducted for 10 minutes to 2 hours at temperature between 20-35 °C.

13. PROCESS FOR GENETIC ENGINEERING OF ACIDOPHILIC BACTERIA according to claim 12, **characterized by** an incubation of cells with permeabilizing agent conducted for 20 minutes to 40 minutes.

14. PROCESS FOR GENETIC ENGINEERING OF ACIDOPHILIC BACTERIA according to claim 8, **characterized by** a washing buffer that is composed of a solution containing 1 mM of magnesium chloride, and 5 mM of sodium phosphate in pH 5.8-7.4

15. PROCESS FOR GENETIC ENGINEERING OF ACIDOPHILIC BACTERIA according to claim 8, **characterized by** a electroporation buffer that is composed of a solution containing 1 mM of sucrose, 3 mM of magnesium chloride and pH 5.8-7.4.

16. PROCESS FOR GENETIC ENGINEERING OF ACIDOPHILIC BACTERIA according to claim 1, **characterized by** comprising the following additional steps: (a) mixture of electrocompetent cells with a constructed vector; (b) application of electric pulses (electroporation); and (c) selection of engineered cells.

17. PROCESS FOR GENETIC ENGINEERING OF ACIDOPHILIC BACTERIA according to claim 16, **characterized by** electric pulses being applied under the following conditions: Capacitance of 25 µF, Resistance of 200Ω, tension around 1.0-2.0 kV for 2.4-5.0 milliseconds.

18. PROCESS FOR GENETIC ENGINEERING OF ACIDOPHILIC BACTERIA according to claim 17, **characterized by** electric pulses applied to a tension between 1.0-1.5 kV.

19. PROCESS FOR GENETIC ENGINEERING OF ACIDOPHILIC BACTERIA according to claim 16, **characterized by** the recovery of electroporated cells in culture media and further selection of engineered cells.

20. TRANSFORMAITON VECTOR whose construction process is composed of the following steps: (1) using pBR322 vector as backbone; (2) containing a vegetative replication origin (Oriv) obtained from commercial transposon; (3) insertion of Oriv in subcloning vector; (4) removal of OriV from determined restriction sites; (5) cloning of OriV in vector pBR322 at the same restriction sites, forming pBR322Oriv vector; (6) digestion of pBR322OriV for inserting the kanamycin-resistant gene. (7) formation of the vector of interest.

21. TRANSFORMATION VECTOR according to claim 20, **characterized by** the use of commercial transposon EZ-Tn5™.

22. TRANSFORMATION VECTOR according to claim 20, **characterized by** the use of commercial subcloning vector pGEM-T.

23. USE OF TRANSFORMATION VECTOR according to any of the claims 20-23, **characterized by** being applied to processes for the transference of gene content to acidophilic bacteria.

24. USE OF ENGINEERED BACTERIA according to any of the claims 1-19, **characterized by** the fact of being applied to the bioleaching processes.
